# EUROPÄISCHE PATENTSCHRIFT

(11) **EP 3 273 457 B1**
(45) Veröffentlichungstag und Bekanntmachung des Hinweises auf die Patenterteilung: **15.01.2020**
(21) Anmeldenummer: 17181858.6
(22) Anmeldetag: 18.07.2017
(51) Int. Cl.: H01H 3/14

(54) **FUSSSCHALTER FÜR EIN MEDIZINISCHES INSTRUMENT**
FOOT-SWITCH FOR A MEDICAL INSTRUMENT
COMMUTATEUR À PIED POUR UN INSTRUMENT MÉDICAL

(30) Priorität: 19.07.2016 DE 102016113259
(43) Veröffentlichungstag der Anmeldung: 24.01.2018
(73) Patentinhaber: Karl Storz SE & Co. KG, 78532 Tuttlingen (DE)
(72) Erfinder: Emmerich, Bernd, 78532 Tuttlingen (DE)

(56) Entgegenhaltungen:
- DE-A1-102013 113 411
- US-A- 5 126 731

## Beschreibung

Die Erfindung betrifft einen Fußschalter für ein medizinisches Instrument mit einem Gehäuse sowie mit einer im Gehäuse angeordneten elastisch verformbaren Saugglocke, wobei zum Festlegen des Gehäuses an einem Untergrund mittels einer Evakuiereinrichtung ein Unterdruck in der Saugglocke erzeugbar ist.

Fußschalter werden in der Medizintechnik häufig verwendet, um Geräte, Kameras und dergleichen zu steuern und verschiedene Funktionen der Geräte zu aktivieren oder zu deaktivieren. Um ein sicheres Betätigen der zu steuernden Geräte zu gewährleisten, ist es zwingend erforderlich, dass der Fußschalter lagefest am Untergrund festlegbar ist.

Ein gattungsgemäßer Fußschalter ist beispielsweise aus der DE 10 2013 113 411 A1 bekannt. Bei diesem bekannten Fußschalter wird der Unterdruck in der Saugglocke durch aktives Absaugen mittels einer elektrisch betriebenen Evakuiervorrichtung erzeugt. Die Verwendung der elektrisch betriebenen Evakuiervorrichtung ist sehr aufwändig.

Davon ausgehend liegt der Erfindung die **Aufgabe** zugrunde, einen Fußschalter für ein medizinisches Instrument zu schaffen, der bei einfachem Aufbau und einfacher Handhabung ein lagefestes Fixieren des Fußschalters an einem Untergrund gewährleistet.

Die **Lösung** dieser Aufgabenstellung ist erfindungsgemäß dadurch gekennzeichnet, dass die Saugglocke zur Erzeugung des Unterdrucks durch Vergrößerung des von der Saugglocke umschlossenen Volumens über die Evakuiereinrichtung elastisch ausdehnbar ist.

Durch die erfindungsgemäße Ausgestaltung der Evakuiereinrichtung, bei der die Saugglocke nicht aktiv evakuiert wird, sondern das umschlossene Volumen vergrößert wird, ist es möglich, ganz auf Ventile zu verzichten, wodurch ein einfacher und kostengünstiger Aufbau möglich ist. Aufgrund der erfindungsgemäßen Ausgestaltung bedarf es folglich auch keiner elektrisch betriebenen Absaugvorrichtung zur Erzeugung des Unterdrucks in der Saugglocke. Durch diese rein mechanische Ausgestaltung ist es auch möglich, den Fußschalter an anderen Oberflächen wie dem Untergrund zu befestigen, beispielsweise an einem medizinischen Gerätewagen zur Aufbewahrung oder zum Transport.

Zudem ist die Evakuiereinrichtung als an der Saugglocke angreifende Zugvorrichtung ausgebildet. Über die Zugvorrichtung wird die Mantelfläche der elastisch verformbaren Saugglocke gedehnt und somit das von der Saugglocke umschlossene Volumen vergrößert, was eine Verringerung des Luftdrucks innerhalb der Saugglocke zur Folge hat, da keine Luft von außen in die Saugglocke nachströmen kann.

Zur Ausgestaltung der Zugvorrichtung wird mit der Erfindung vorgeschlagen, dass die Zugvorrichtung als Zugstab ausgebildet ist, der über einen außen am Gehäuse gelagerten Kipphebel betätigbar ist.

Der Kipphebel ist gemäß einer bevorzugten Ausführungsform der Erfindung als zweiarmiger Kipphebel ausgebildet, dessen Arme zwischen sich einen Winkel von 90° bis 140° aufspannen und der im Kontaktpunkt der beiden Arme verschwenkbar auf einer Schwenkachse am Gehäuse gelagert ist. Dieser am Gehäuse angeordnete Kipphebel lässt sich auf einfache Art und Weise entweder mit einem Fuß oder einer Hand betätigen, um durch die Erzeugung des Unterdrucks in der Saugglocke das Gehäuse des Fußschalters an einem Untergrund lagefest zu fixieren. Neben der Fixierung des Fußschalters am Fußboden ist es zu Transport- und/oder Lagerungszwecken ebenfalls möglich, den Fußschalter über die Saugglocke an einem Gerätewagen zu befestigen.

Weiterhin wird mit der Erfindung vorgeschlagen, dass der Kipphebel über einen ersten Arm an der Zugvorrichtung gelagert ist und das der Kipphebel über einen zweiten Arm zwischen einer die Zugvorrichtung freigebenden und die Saugglocke entspannenden Grundposition und einer die Saugglocke über die Zugvorrichtung dehnenden Saugposition verschwenkbar ist. Das Verschwenken des Kipphebels hat somit eine direkte Auswirkung auf die Saugglocke und somit auf das Fixieren oder Lösen des Fußschalters in Bezug auf dessen Aufstandsfläche.

Um ein versehentliches Betätigen des sich in der Saugposition befindlichen Kipphebels und somit ein versehentliches Lösen des Fußschalters von der Untergrundfläche zu verhindern, wird mit der Erfindung vorgeschlagen, dass der zweite Arm des Kipphebels in der die Zugvorrichtung betätigenden Saugposition am Gehäuse fixierbar ist.

Gemäß einer ersten praktischen Ausführungsform der Erfindung erfolgt das Fixieren des zweiten Arms des Kipphebels am Gehäuse in der Saugposition über einen am Gehäuse oder am zweiten Arm des Kipphebels angeordneten Magneten. Die Verwendung eines Magneten zum Fixieren des Kipphebels in der Saugposition stellt eine einfache und kostengünstige Bauweise dar, die darüber hinaus gut zu reinigen ist.

Mit einer zweiten Ausführungsform wird erfindungsgemäß vorgeschlagen, dass die Schwenkachse durch eine mit mindestens einer planen Abflachung versehene Kugel verläuft, auf der der Kipphebel gelagert ist. Ferner wird vorgeschlagen, dass das Fixieren des Kipphebels am Gehäuse in der Saugposition über mindestens ein Federelement erfolgt, das auf die mindestens eine plane Abflachung der Kugel einwirkt.

Alternativ ist es selbstverständlich auch möglich, den zweiten Arm des Kipphebels in Saugposition über einen Klemmmechanismus am Gehäuse zu fixieren.

Schließlich wird mit der Erfindung vorgeschlagen, dass die Saugglocke über eine Ventileinrichtung verfügt und über diese belüftbar ist, um das Lösen der Saugglocke vom Untergrund zu erleichtern. Das Ventil verbindet die Innenseite der Saugglocke mit der Umgebung. Das Ventil kann bei Bedarf geöffnet und wieder geschlossen werden.

Weitere Merkmale und Vorteile der Erfindung ergeben sich anhand der zugehörigen Zeichnungen, in denen zwei Ausführungsbeispiele eines erfindungsgemäßen Fußschalters für ein medizinisches Instrument nur beispielhaft dargestellt sind, ohne die Erfindung auf diese Ausführungsbeispiele zu beschränken. In den Zeichnungen zeigt:
- Fig. 1: einen schematischen Längsschnitt durch einen erfindungsgemäßen Fußschalter für ein medizinisches Instrument;
- Fig. 2: eine schematische Draufsicht auf eine Saugglocke;
- Fig. 3: eine vergrößerte Darstellung des Details III gemäß Fig. 1, eine erste Ausführungsform des Kipphebels darstellend und
- Fig. 4: eine Abbildung gemäß Fig. 3, jedoch eine zweite Ausführungsform des Kipphebels darstellend.

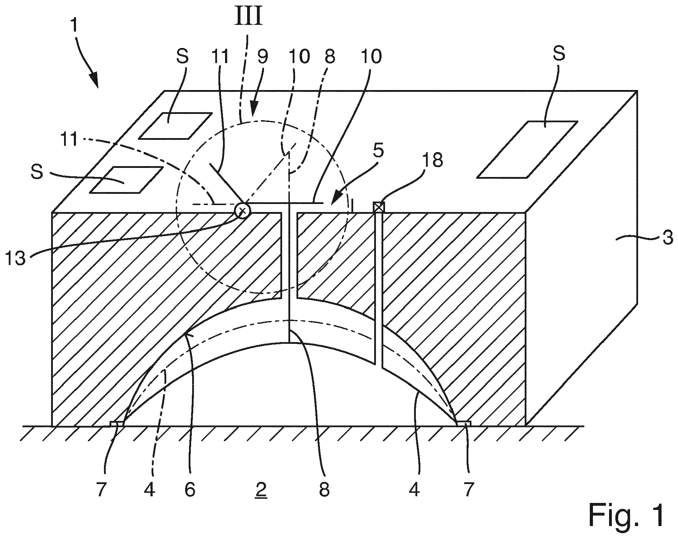

Die Abbildung Fig. 1 zeigt schematisch einen Längsschnitt durch einen Fußschalter 1 für ein nicht dargestelltes medizinisches Instrument. Fußschalter 1 werden in der Medizintechnik häufig verwendet, um medizinische Instrumente, Geräte, Kameras und dergleichen zu steuern und verschiedene Funktionen der medizinischen Instrumente zu aktivieren oder zu deaktivieren.

Um ein sicheres Betätigen der zu steuernden Instrumente zu gewährleisten, ist es zwingend erforderlich, dass der Fußschalter 1 lagefest an einem Untergrund 2 festlegbar ist. Hierfür hat es sich in der Praxis bewährt, in einem Gehäuse 3 des Fußschalters 1 eine elastisch verformbare Saugglocke 4 anzuordnen, wobei zum Festlegen des Gehäuses 3 am Untergrund 2 mittels einer Evakuiereinrichtung 5 ein Unterdruck in der Saugglocke 4 erzeugbar ist.

Durch das Erzeugen des Unterdrucks in der im Gehäuse 3 angeordneten Saugglocke 4 wird das Gehäuse 3 des Fußschalters 1 durch den auf das Gehäuse 3 einwirkenden höheren äußeren Atmosphärendruck lagefest auf den Untergrund 2 gedrückt.

Der Unterdruck in der Saugglocke 4 kann einerseits dadurch erzeugt werden, dass mittels einer Evakuiereinrichtung aktiv Luft aus der Saugglocke 4 herausgesaugt wird, oder aber dadurch, dass das von der Saugglocke 4 umschlossene Volumen vergrößert wird, was eine Verringerung des Luftdrucks innerhalb der Saugglocke 4 zur Folge hat, wenn keine Luft von außen in die Saugglocke 4 nachströmen kann.

Der in Fig. 1 dargestellte Fußschalter 1 für ein medizinisches Instrument besteht im Wesentlichen aus dem Gehäuse 3, in dem ein zum Untergrund 2 hin offener Aufnahmeraum 6 zur Aufnahme der Saugglocke 4 ausgebildet ist. Zum Bedienen des über den Fußschalter 1 steuerbaren medizinischen Instruments sind auf der Oberseite des Gehäuses verschiedene, nicht näher bezeichnete Schalter S angeordnet.

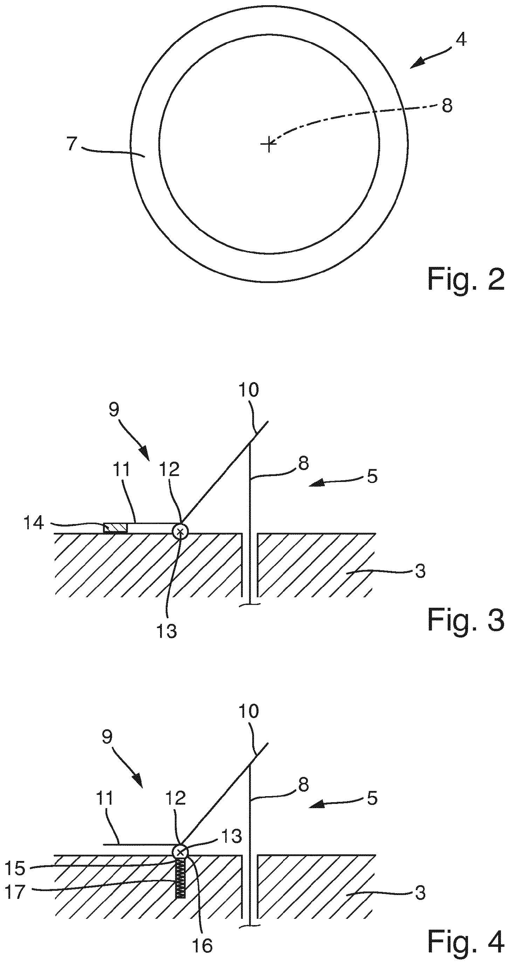

Wie aus Fig. 1 und 2 ersichtlich, weist die halbkugelförmig ausgebildete Saugglocke 4 einen als Dichtlippe gegenüber dem Gehäuse 3 wirkenden umlaufenden Rand 7 auf. Die Saugglocke 4 besteht aus einem elastisch verformbaren Material, wie beispielsweise Gummi oder Silikon.

Das Erzeugen des zum Festlegen des Gehäuses 3 am Untergrund 2 erforderlichen Unterdrucks erfolgt bei der dargestellten Ausführungsform über die als Zugvorrichtung 8 ausgebildete Evakuiereinrichtung 5. Hierzu ist die Zugvorrichtung 8 als an der Saugglocke 4 angreifender Zugstab ausgebildet, über den durch Ausüben einer Zugkraft auf die Mantelfläche der Saugglocke 4 das von der Saugglocke 4 umschlossene Volumen vergrößert wird. Da durch den unter dem Gehäuse 3 angeordneten abdichtenden umlaufenden Rand 7 der Saugglocke 4 keine Luft von außen in die Saugglocke 4 nachströmen kann, bewirkt dieses Ausdehnen der Saugglocke 4 über die Zugvorrichtung 8 die Erzeugung eines Unterdrucks innerhalb der Saugglocke 4.

Das Betätigen der Zugvorrichtung 8 erfolgt bei der dargestellten Ausführungsform über einen außen am Gehäuse 3 angeordneten Kipphebel 9, der als zweiarmiger Kipphebel 9 ausgebildet ist, dessen Arme 10 und 11 zwischen sich einen Winkel von 90° bis 140° aufspannen und der in einem Kontaktpunkt 12 der beiden Arme 10 und 11 verschwenkbar auf einer Schwenkachse 13 am Gehäuse 3 gelagert ist.

Wie weiterhin aus Fig. 1 ersichtlich, ist der Kipphebel 9 über den ersten Arm 10 an der Zugvorrichtung 8 gelagert und über zweiten Arm 11 zwischen einer die Zugvorrichtung 8 freigebenden und die Saugglocke 4 entspannenden Grundposition und einer die Saugglocke 4 über die Zugvorrichtung 8 dehnenden Saugposition verschwenkbar.

In Fig. 1 ist die die Zugvorrichtung 8 freigebende und die Saugglocke 4 entspannenden Grundposition mit durchgezogener Linie dargestellt, wohingegen die die Saugglocke 4 über die Zugvorrichtung 8 dehnende Saugposition und die zugehörige Lage des Kipphebels 9 strichpunktiert dargestellt ist.

Um zu gewährleisten, dass beim Verschwenken des Kipphebels 9 über den Zugstab der Zugvorrichtung 8 eine im Wesentlichen vertikale Zugkraft auf die Saugglocke 4 ausgeübt wird, ist der Zugstab der Zugvorrichtung 8 sowohl an der Saugglocke 4 als auch am ersten Arm 10 des Kipphebels 9 gelenkig gelagert.

Das Lösen des Fußschalters 1 vom Untergrund 2 erfolgt dadurch, dass der Kipphebel 9 wieder zurück verschwenkt wird. Durch die Elastizität des Materials der Saugglocke 4 zieht sich die Saugglocke 4 wieder in ihre ursprüngliche Form zusammen, sobald über die Zugvorrichtung 8 keine Zugkraft mehr auf die Saugglocke 4 ausgeübt wird.

Das Zusammenziehen der Saugglocke 4 bewirkt das Ansteigen des Innendrucks innerhalb der Saugglocke 4 auf den anfänglichen Atmosphärendruck. Jetzt lässt sich der Fußschalter 1 wieder leicht vom Untergrund 2 abheben.

Um ein versehentliches Betätigen des sich in der Saugposition befindlichen Kipphebels 9 und somit ein versehentliches Lösen des Fußschalters 1 vom Untergrund 2 zu verhindern, ist der zweite Arm 11 des Kipphebels 9 in der die Zugvorrichtung 8 betätigenden Saugposition am Gehäuse 3 fixierbar.

In den Abbildungen Fig. 3 und 4 sind zwei Ausführungsbeispiele dargestellt, wie der zweite Arm 11 des Kipphebels 9 in der Saugposition am Gehäuse 3 fixierbar ist.

Bei der in Fig. 3 dargestellten ersten Ausführungsform erfolgt das Fixieren des zweiten Arms 11 des Kipphebels 9 am Gehäuse 3 in der Saugposition über einen am zweiten Arm 11 des Kipphebels 9 angeordneten Magneten 14, der mit einer magnetischen Reaktionsfläche am Gehäuse 3 zusammenwirkt. Alternativ kann der Magnet 14 auch am oder im Gehäuse 3 angeordnete sein und mit einer magnetischen Reaktionsfläche am zweiten Arm 11 des Kipphebels 9 zusammenwirken.

Gemäß der in Fig. 4 dargestellten zweiten Ausführungsform verläuft die Schwenkachse 13, um die der Kipphebel 9 verschwenkbar ist, durch eine mit mindestens einer planen Abflachung 15 versehene Kugel 16, auf der der Kipphebel 9 gelagert ist. Das Fixieren des Kipphebels 9 am Gehäuse 3 in der Saugposition erfolgt bei dieser Ausführungsform über mindestens ein Federelement 17, das auf die mindestens eine plane Abflachung 15 der Kugel 16 einwirkt.

Wie aus Fig. 4 ersichtlich, ist das als Druckfeder ausgebildete Federelement 17 so im Gehäuse 3 gelagert, dass das Gehäuse 3 an einem Ende des Federelements 17 ein Widerlager für das Federelement 17 bildet und das freie andere Ende des Federelements 17 an der Kugel 16 anliegt.

In der die Zugvorrichtung 8 freigebenden und die Saugglocke 4 entspannenden Grundposition liegt das freie Ende des Federelements 17 an der kugelförmigen äußeren Oberfläche der Kugel 16 an. Die Federkraft des Federelements 17 ist so bemessen, dass das Federelement 17 bei der Anlage an der kugelförmigen äußeren Oberfläche der Kugel 16 das Verschwenken des Kipphebels 9 nicht hemmt.

In der verschwenkten Saugposition des Kipphebels 9 liegt das freie Ende des Federelements 17 nunmehr nicht mehr an der kugelförmigen äußeren Oberfläche der Kugel 16, sondern an der planen Abflachung 15 der Kugel 16 an. Auf diese plane Abflachung 15 der Kugel 16 übt das Federelement 17 eine derartige Druckkraft aus, dass ein versehentliches Betätigen des sich in der Saugposition befindlichen Kipphebels 9 und somit ein versehentliches Lösen des Fußschalters 1 vom Untergrund 2 ausgeschlossen ist.

Zum Überführen des Kipphebels 9 zurück in die die Zugvorrichtung 8 freigebende und die Saugglocke 4 entspannende Grundposition bedarf es einer gezielten Kraftausübung auf den Kipphebel 9, um diesen gegen den vom Federelement 17 auf die Abflachung 15 der Kugel 16 ausgeübten Anpressdruck zu verschwenken.

Da Saugglocken 4 dazu neigen können, auch nach dem Abschalten des Unterdrucks, also nach dem Zurückschwenken des Kipphebels 9 in die Grundposition und dem Zusammenziehen der Saugglocke 4, über den als Dichtlippe wirkenden umlaufenden Rand 7 weiter am Untergrund 2 festgesaugt zu bleiben, weist die Ausführungsform gemäß Fig. 1 eine zusätzliche Ventileinrichtung 18 auf, über die die Saugglocke 4 nach dem Aufheben des Unterdrucks zusätzlich von außen belüftbar ist.

Ein wie voranstehend beschrieben aufgebauter Fußschalter 1 für ein medizinisches Instrument zeichnet sich dadurch aus, dass bei einfachem Aufbau und einfacher Handhabung ein lagefestes Fixieren des Fußschalters 1 an einem Untergrund 2 gewährleistet ist.

### Bezugszeichenliste

- 1: Fußschalter
- 2: Untergrund
- 3: Gehäuse
- 4: Saugglocke
- 5: Evakuiereinrichtung
- 6: Aufnahmeraum
- 7: Rand
- 8: Zugvorrichtung
- 9: Kipphebel
- 10: erster Arm
- 11: zweiter Arm
- 12: Kontaktpunkt
- 13: Schwenkachse
- 14: Magnet
- 15: Abflachung
- 16: Kugel
- 17: Federelement
- 18: Ventileinrichtung

- S: Schalter

## Patentansprüche

1. Fußschalter für ein medizinisches Instrument mit einem Gehäuse (3) sowie mit einer im Gehäuse (3) angeordneten elastisch verformbaren Saugglocke (4), wobei zum Festlegen des Gehäuses (3) an einem Untergrund (2) mittels einer Evakuiereinrichtung (5) ein Unterdruck in der Saugglocke (4) erzeugbar ist,
wobei die Saugglocke (4) zur Erzeugung des Unterdrucks durch Vergrößerung des von der Saugglocke (4) umschlossenen Volumens über die Evakuiereinrichtung (5) elastisch ausdehnbar ist, **dadurch gekennzeichnet, dass** die Evakuiereinrichtung (5) als an der Saugglocke (4) angreifende Zugvorrichtung (8) ausgebildet ist.

2. Fußschalter nach Anspruch 1, **dadurch gekennzeichnet, dass** die Zugvorrichtung (8) als Zugstab ausgebildet ist, der über einen außen am Gehäuse (3) gelagerten Kipphebel (9) betätigbar ist.

3. Fußschalter nach Anspruch 2, **dadurch gekennzeichnet, dass** der Kipphebel (9) als zweiarmiger Kipphebel (9) ausgebildet ist, dessen Arme (10, 11) zwischen sich einen Winkel von 90° bis 140° aufspannen und der im Kontaktpunkt (12) der beiden Arme (10, 11) verschwenkbar auf einer Schwenkachse (13) am Gehäuse (3) gelagert ist.

4. Fußschalter nach Anspruch 3, **dadurch gekennzeichnet, dass** der Kipphebel (9) über einen ersten Arm (10) an der Zugvorrichtung (8) gelagert ist und das der Kipphebel (9) über einen zweiten Arm (11) zwischen einer die Zugvorrichtung (8) freigebenden und die Saugglocke (4) entspannenden Grundposition und einer die Saugglocke (4) über die Zugvorrichtung (8) dehnenden Saugposition verschwenkbar ist.

5. Fußschalter nach Anspruch 4, **dadurch gekennzeichnet, dass** der zweite Arm (11) des Kipphebels (9) in der die Zugvorrichtung (8) betätigenden Saugposition am Gehäuse fixierbar ist.

6. Fußschalter nach Anspruch 5, **dadurch gekennzeichnet, dass** das Fixieren des zweiten Arms (11) des Kipphebels (9) am Gehäuse (3) in der Saugposition über einen am Gehäuse (3) oder am zweiten Arm (11) des Kipphebels (9) angeordneten Magneten (14) erfolgt.

7. Fußschalter nach einem der Ansprüche 3 bis 5, **dadurch gekennzeichnet, dass** die Schwenkachse (13) durch eine mit mindestens einer planen Abflachung (15) versehene Kugel (16) verläuft, auf der der Kipphebel (9) gelagert ist.

8. Fußschalter nach Anspruch 7, **dadurch gekennzeichnet, dass** das Fixieren des Kipphebels (9) am Gehäuse (3) in der Saugposition über mindestens ein Federelement (17) erfolgt, das auf die mindestens eine plane Abflachung (15) der Kugel (16) einwirkt.

9. Fußschalter nach einem der Ansprüche 1 bis 8, **dadurch gekennzeichnet, dass** die Saugglocke (4) über eine Ventileinrichtung (18) belüftbar ist.

## Claims

1. Footswitch for a medical instrument, with a housing (3), and with an elastically deformable suction cup (4) arranged in the housing (3), it being possible for a negative pressure to be generated in the suction cup (4) by means of an evacuation device (5) in order to secure the housing (3) to a floor surface (2),
wherein the suction cup (4) is elastically extensible via the evacuation device (5) in order to generate the negative pressure by enlargement of the volume enclosed by the suction cup (4), **characterized in that** the evacuation device (5) is designed as a tensioning device (8) engaging on the suction cup (4).

2. Footswitch according to Claim 1, **characterized in that** the tensioning device (8) is designed as a tension rod which can be actuated via a toggle lever (9) mounted externally on the housing (3).

3. Footswitch according to Claim 2, **characterized in that** the toggle lever (9) is designed as a two-armed toggle lever (9), of which the arms (10, 11) between them span an angle of 90° to 140°, and is mounted pivotably at a pivot axis (13) on the housing (3) at the contact point (12) of the two arms (10, 11).

4. Footswitch according to Claim 3, **characterized in that** the toggle lever (9) is mounted on the tensioning device (8) via a first arm (10), and the toggle lever (9) is pivotable, via a second arm (11), between a starting position, in which the tensioning device (8) is released and the suction cup (4) untensioned, and a suction position, in which the suction cup (4) is extended via the tensioning device (8).

5. Footswitch according to Claim 4, **characterized in that** the second arm (11) of the toggle lever (9) can be fixed to the housing in the suction position actuating the tensioning device (8).

6. Footswitch according to Claim 5, **characterized in that** the fixing of the second arm (11) of the toggle lever (9) to the housing (3) in the suction position is effected via a magnet (14) arranged on the housing (3) or on the second arm (11) of the toggle lever (9).

7. Footswitch according to one of Claims 3 to 5, **characterized in that** the pivot axis (13) extends through a ball (16), which is provided with at least one plane flattening (15) and on which the toggle lever (9) is mounted.

8. Footswitch according to Claim 7, **characterized in that** the fixing of the toggle lever (9) to the housing (3) in the suction position is effected via at least one spring element (17), which acts on the at least one plane flattening (15) of the ball (16).

9. Footswitch according to one of Claims 1 to 8, **characterized in that** the suction cup (4) can be ventilated via a valve device (18).

## Revendications

1. Interrupteur à pédale pour un instrument médical doté d'un boîtier (3) ainsi que d'une ventouse (4) élastiquement déformable agencée dans le boîtier (3), dans lequel une dépression peut être produite dans la ventouse (4) au moyen d'un dispositif d'évacuation (5) afin de fixer le boîtier (3) à un sol (2),
dans lequel, afin de produire la dépression, la ventouse (4) est élastiquement dilatable par agrandissement du volume entouré par la ventouse (4) par le biais du dispositif d'évacuation (5), **caractérisé en ce que** le dispositif d'évacuation (5) est réalisé comme dispositif de traction (8) venant en prise sur la ventouse (4).

2. Interrupteur à pédale selon la revendication 1, **caractérisé en ce que** le dispositif de traction (8) est réalisé comme tirant, lequel est actionnable par le biais d'un levier basculant (9) disposé à l'extérieur sur le boîtier (3).

3. Interrupteur à pédale selon la revendication 2, **caractérisé en ce que** le levier basculant (9) est réalisé comme levier basculant à deux bras (9), dont les bras (10, 11) décrivent entre eux un angle de 90° à 140° et lequel est logé sur le boîtier (3) en pivotement sur un axe de pivot (13) dans le point de contact (12) des deux bras (10, 11).

4. Interrupteur à pédale selon la revendication 3, **caractérisé en ce que** le levier basculant (9) est posé sur le dispositif de traction (8) par le biais d'un premier bras (10) et **en ce que** le levier basculant (9) peut pivoter par le biais d'un deuxième bras (11) entre une position de base libérant le dispositif de traction (8) et détendant la ventouse (4) et une position d'aspiration étirant la ventouse (4) par le biais du dispositif de traction (8).

5. Interrupteur à pédale selon la revendication 4, **caractérisé en ce que** le deuxième bras (11) du levier basculant (9) peut être bloqué au niveau du boîtier dans la position d'aspiration actionnant le dispositif de traction (8).

6. Interrupteur à pédale selon la revendication 5, **caractérisé en ce que** le blocage du deuxième bras (11) du levier basculant (9) dans la position d'aspiration sur le boîtier (3) s'effectue par le biais d'un aimant (14) agencé sur le boîtier (3) ou sur le deuxième bras (11) du levier basculant (9).

7. Interrupteur à pédale selon l'une des revendications 3 à 5, **caractérisé en ce que** l'axe de pivot (13) traverse une boule (16) prévue dotée d'au moins un aplatissement plan (15), sur laquelle est posée le levier basculant (9).

8. Interrupteur à pédale selon la revendication 7, **caractérisé en ce que** le blocage du levier basculant (9) sur le boîtier (3) dans la position d'aspiration s'effectue par le biais d'au moins un élément de ressort (17), lequel opère sur l'au moins un aplatissement plan (15) de la boule (16).

9. Interrupteur à pédale selon l'une des revendications 1 à 8, **caractérisé en ce que** la ventouse (4) est ventilable par le biais d'un moyen de soupape (18).
